# DEMANDE DE BREVET EUROPEEN

(11) **EP 1 358 867 A1**
(43) Date de publication de la demande: **05.11.2003**
(21) Numéro de dépôt: 03290939.2
(22) Date de dépôt: 16.04.2003
(51) Int. Cl.: A61K 7/06, A61K 7/48

(54) **Composition cosmétique comprenant un composé biotine et/ou un agent complexant**

(30) Priorité: 19.04.2002 FR 0204952
(71) Demandeur: L'OREAL, 75008 Paris (FR)
(72) Inventeur: Vic, Gabin, 60200 Compiegne (FR); Livoreil, Aude, 75006 Paris (FR); Bernard, Bruno, 92200 Neuilly Sur Seine (FR)
(74) Mandataire: Casalonga, Axel

(57) **Abrégé**

La présente invention est relative à des compositions cosmétiques comprenant un composé biotine et/ou un agent complexant, l'un et/ou l'autre étant porteur d'au moins une fonction chimique réactive permettant une fixation covalente aux cheveux et fixé de façon covalente à l'un de ces deux composés ou au deux, au moins un actif cosmétique.

La présente invention est également relative à un procédé de traitement du cheveu à base de ces compositions, visant à créer sur la fibre un dépôt de matière apportant de nouvelles propriétés cosmétiques.

## Description

La présente invention est relative à une composition cosmétique comprenant au moins un composé biotine (biotine modifiée ou non) porteur d'au moins un groupement d'ancrage α permettant une fixation covalente aux cheveux et éventuellement porteur d'au moins un groupement à activité cosmétique.

La présente invention concerne aussi une composition cosmétique comprenant au moins un agent complexant du composé biotine (biotine modifiée ou non), porteur d'au moins un groupement d'ancrage α' permettant une fixation covalente aux cheveux et porteur éventuellement d'au moins un groupement à activité cosmétique.

La présente invention a encore pour objet une composition cosmétique comprenant un composé biotine (biotine modifiée ou non) porteur d'au moins un groupement d'ancrage α permettant une fixation covalente aux cheveux associé à au moins un agent complexant, le composé biotine ou l'agent complexant étant porteur d'au moins un groupement à activité cosmétique.

La présente invention est encore relative à une composition cosmétique comprenant un agent complexant porteur d'au moins un groupement d'ancrage α' permettant une fixation covalente aux cheveux associé à au moins un composé biotine, le composé biotine ou l'agent complexant étant porteur d'au moins un groupement à activité cosmétique.

La présente invention concerne une composition cosmétique comprenant un agent complexant porteur d'au moins un groupement d'ancrage α' permettant une fixation covalente aux cheveux associé à au moins un composé biotine (biotine modifiée ou non) porteur d'au moins un groupement d'ancrage α permettant une fixation covalente aux cheveux, le composé biotine et/ou l'agent complexant étant porteur d'au moins un groupement à activité cosmétique.

La présente invention a également pour objet des procédés de traitement du cheveu à base de ces composés, visant à créer sur la fibre un dépôt de matière fixé de façon covalente apportant de nouvelles propriétés cosmétiques.

Il est intéressant de pouvoir fixer sur les matières kératiniques et en particulier les cheveux, des actifs apportant un bénéfice de façon durable. Pour atteindre cet objectif, le greffage direct par voie chimique constitue une solution possible. Malheureusement, ces techniques de greffage chimique posent des problèmes d'innocuité ou conduisent à une dégradation de l'intégrité des matières kératiniques. De plus, ces techniques de greffage sont souvent irréversibles dans un contexte cosmétique. Par ailleurs, la simple adsorption d'actifs sur la fibre donnent des effets qui s'éliminent au premier shampoing. Il subsiste donc un besoin de créer un dépôt de matière sur le cheveu qui soit rémanent aux shampooings, ne dégradant pas la fibre capillaire qui soit démaquillable à souhait, et qui soit éventuellement réticulable.

La biotine, appelée aussi vitamine H ou B8, est une vitamine hydrosoluble constituée d'un noyau imidazoline et d'un cycle tétrahydrothiophène porteur d'une chaîne latérale à cinq atomes de carbone. La biotine est un élément nutritif des cheveux et de la peau. La biotine se fixe naturellement avec une très grande affinité à l'avidine, glycoprotéine que l'on trouve dans le blanc d'oeuf cru. La biotine et l'avidine forment ensemble un complexe par liaison non-covalente parmi les plus stables existant entre des molécules biologiques avec une constante de dissociation de 1,3 10⁻¹⁵ à pH5. De plus, l'avidine possède quatre sites d'interaction pour la biotine.

L'invention a donc pour objet une composition cosmétique comprenant un composé biotine a) et un agent complexant b) susceptible de former un complexe avec le composé biotine de constante de dissociation inférieure ou égale à 10⁻¹ à la température de 25C°, le composé biotine étant lié à un groupement d'ancrage α et/ou l'agent complexant étant lié à un groupement d'ancrage α', les groupements α et α' permettant une fixation covalente aux cheveux et le composé biotine et/ou l'agent complexant étant porteur d'au moins un groupement à activité cosmétique.

Un autre objet de l'invention est l'ensemble des procédés de traitement cosmétique des matières kératiniques décrits ci-après.

D'autres objets de la présente demande ressortiront à la lecture de la description et des exemples qui suivent.

Au sens de la présente invention, un groupement d'ancrage α ou α' est un groupement chimique qui permet la fixation covalente du composé portant ledit groupement sur les fibres kératiniques sous l'action d'un stimulus autre que les ondes électromagnétiques de longueur d'onde comprises entre 200 et 800 nm. Les stimuli utilisables selon la présente invention sont par exemple l'agitation moléculaire, la chaleur, le pH, les catalyseurs organiques et/ou minéraux, l'oxygène, les ultrasons.

Les composés a) c'est-à-dire la biotine et les biotines modifiées sont de préférence choisis parmi les composés de formule **(I)** 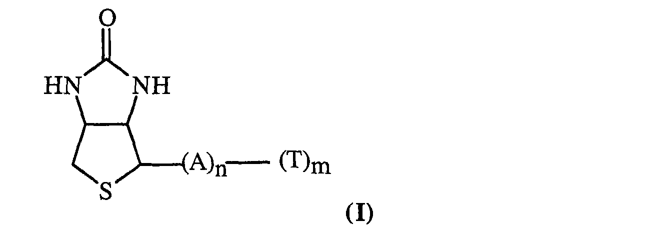
- avec A désignant un radical divalent comprenant de 1 à 100 atomes de carbone, linéaire ou ramifié, saturé ou non, de préférence alkylène de 1 à 30 atomes de carbone, interrompu ou non par un ou plusieurs hétéroatomes et éventuellement substitué par un ou plusieurs groupements choisis parmi les groupements hydroxyle, amino, halogènes, aryle, phosphate, phosphorate, sulfate, sulfonate, carboxy, alcoxycarbonyle, alcoxy.
- avec T désignant un groupement α ou un radical BW(α)p W étant un résidu à activité cosmétique et B une entité de fixation du résidu W au squelette dérivé de biotine,
- α désignant un groupement d'ancrage
- avec n désignant 0 ou 1
- avec m désignant un nombre allant de 1 à 100
- avec p désignant un nombre allant de 0 à 100
- avec B désignant par exemple un groupement amine, imine, amide, ester, disulfure, thioester, uréthane, urée, éther, thioéther, azoïque, méthine, époxy, un résidu aromatique ou hétérocyclique, dialkylpolysiloxane.
- avec W pouvant être issu de molécules filtrant les UV, de molécules hydratantes ou émollientes, d'agents conditionneurs, d'agents antistatiques, d'agents antitranspirants, de matières parfumantes, de molécules réductrices, de molécules oxydantes, d'agents antimicrobiens, d'agents antipelliculaires, d'agents particulaires ayant des propriétés de maintien, de photoprotection et/ou de coloration sous forme de particules minérales ou organiques éventuellement sous forme de nanotubes ou d'autres assemblages tridimensionnels comme les fullérènes (ex. fullérènes C60-C70) sur lesquels sont éventuellement adsorbés un ou plusieurs polymères, d'agents filmogènes anioniques ou non ioniques, amphotères ou cationiques sur lesquels sont éventuellement adsorbées des particules organiques ou minérales.

Les dérivés de biotine utilisables selon l'invention sont par exemple disponibles chez Sigma-Aldrich ou Carbomer Inc et peuvent être choisis parmi l'albumine bovine biotine, l'albumine bovine biotine gold labelled (5 ou 20 nm), la biotine dextrane, la biotine fluoresceine, le latex biotine, la biotine carboxyméthylcellulose, la biotine chitosane, la peroxidase biotinamidocaproyl, la phosphatase alkaline-biotinamidocaproyl, la biotine agarose, la concanavaline A-biotine, la β-galactosidase-biotine, les conjugués lectines-biotine, la B-phycoerythrine-biotine.

Le groupement d'ancrage α est choisi de préférence parmi ceux de la liste suivante :
thiosulfates ; aldéhydes ; époxides ; alkoxysilane ; silanol ; aziridine ; acétal ; hémi-acétal ; aminal ; hémi-aminal ; oxazine et oxazoline ; oxazinium et oxazolinium ; vinyle et vinyle activé : acrylonitrite, esters acrylique et métacrylique, acide et esters crotonique, acides et esters cinnamique, styrène et dérivés, butadiène, éthers de vinyle, vinylacétone, esters maléiques, vinyl sulfones, maléimides ; cétones et alpha-hydroxycétones, alpha-halocétones ; halogénures d'alkyle ou d'aryle, ou aryalkyle RX (X=I, Br, Cl) ; halogénure de cycle insaturé (cycle carboné ou hétérocycle) RX :chlorotriazine, chloropyrimidine, chlorochinoxaline, chlorobenzotriazole ; halogénure de sulfonyle : RSO₂Cl ou F ;(dans les 3 derniers cas d'autres groupes portants X peuvent être aussi : OSO3H, N(Me)3, SO2Me, OPO3H, SO2Et) para-nitrophényl ester ; siloxane ; halogénosilanes, alcoxysilanes ; halogénoacétates ; hydracides ; difluorodinitrobenzène.

Les polymères susceptibles soit d'être adsorbés sur les particules ou sur lesquels sont éventuellement adsorbés les particules sont choisis parmi les polymères cationiques, amphotères, non ioniques ou anioniques définis ci-dessus en relation avec W.

Le groupement W peut être issu des actifs suivants :
les saccharides, les oligosaccharides, les polysaccharides hydrolysés ou non, modifiés ou non,
les acides aminés, les oligopeptides, les peptides, les protéines hydrolysées ou non, modifiées ou non, les polyacides aminés, les enzymes,
les acides et alcools gras ramifiés ou non,
les cires animales, végétales ou minérales,
les céramides et les pseudo-céramides,
les acides organiques hydroxylés,
les filtres UV,
les antioxydants et les agents anti-radicaux libres,
les chélatants,
les agents antipelliculaires,
les agents régulateurs de séborrée,
les agents apaisants,
les tensioactifs cationiques,
les polymères cationiquesou amphotères,
les silicones organomodifiées ou non,
les huiles minérales, végétales ou animales,
les polyisobutènes et poly(α-oléfines),
les esters,
les polymères anioniques solubles ou dispersés,
les polymères non-ioniques solubles ou dispersés,
les agents réducteurs
les colorants ou les pigments
et leurs mélanges.

D'une manière générale, les composés de type saccharides, oligosaccharides, polysaccharides hydrolysés ou non, modifiés ou non, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

A titre d'exemples de saccharides, oligosaccharides, polysaccharides hydrolysés ou non, modifiés ou non, utilisables dans l'invention, on peut notamment citer les glucanes, les amidons modifiés ou non (tels que ceux issus, par exemple, de céréales comme le blé, le maïs ou le riz, de légumes comme le pois blond, de tubercules comme les pommes de terre ou le manioc) et différents du bétaïnate d'amidon tel que décrit ci-dessus, l'amylose, l'amylopectine, le glycogène les dextranes, les β-glucanes, les celluloses et leurs dérivés (méthylcelluloses, hydroxyalkylcelluloses, éthylhydroxyéthylcelluloses, carboxyméthyl-celluloses), les fructosanes, l'inuline, la levane, les mannanes, les xylanes, les lignines, les arabanes, les galactanes, les galacturonanes, la chitine, les glucoronoxylanes, les arabinoxylanes, les xyloglucanes, les galactomannanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les gommes de guar et les gommes de xanthane.

Comme acides aminés, on peut citer, par exemple, la cystéine, la lysine, l'alanine, la N-phénylalanine, l'arginine, la glycine, la leucine, et leurs mélanges. A titre d'oligopeptides, de peptides, de protéines hydrolysées ou non, modifiées ou non, que l'on peut utiliser selon l'invention, on peut notamment citer les hydrolysats de protéines de laine ou de soie, modifiées ou non, les protéines végétales telles que les protéines de blé.

Parmi les polyacides aminés utilisables, on peut citer la polylysine.

Parmi les enzymes utilisables, on peut citer les laccases, les peroxydases, les lipases, les protéases, les glycosidases, les dextranases, les uricases, la phosphatase alcaline.

Parmi les acides gras ramifiés ou non convenant dans la présente invention, on peut notamment citer les acides carboxyliques en C₈-C₃₀, tels que l'acide palmitique, l'acide oléique, l'acide linoléique, l'acide myristique, l'acide stéarique, l'acide laurique, et leurs mélanges. Les alcools gras utilisables dans la présente invention, comprennent notamment les alcools en C₈-C₃₀ comme, par exemple, les alcools palmitylique, oléylique, linoléylique, myristylique, stéarylique et laurylique.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines ou les ozokérites.

Parmi les céramides, on peut citer en particulier les céramides des classes I, II, III et V selon la classification de DAWNING, et plus particulièrement la N-oléyldéshydrosphingosine.

Les acides organiques hydroxylés sont choisis parmi ceux bien connus et utilisés dans la technique. On peut notamment citer l'acide citrique, l'acide lactique, l'acide tartrique, l'acide malique.

Les filtres solaires actifs dans l'UV-A et/ou l'UV-B utilisables selon l'invention sont ceux bien connus de l'homme de métier. On peut notamment citer les dérivés du dibenzoylméthane tels que le 4-méthyldibenzoylméthane, le 4-isopropyldibenzoylméthane, le 4-tert-butyldibenzoylméthane, le 2,4-diméthyldibenzoylméthane, le 4-tert-butyl-4'-diisopropyldibenzoyl-méthane, l'acide p-amino-benzoïque et ses esters tels que le p-diméthylaminobenzoate de 2-éthylhexyle et le p-aminobenzoate d'éthyle N-propoxylé, les salicylates tels que le salicylate de triéthanolamine, les esters d'acide cinnamique tels que le 4-méthoxy-cinnamate de 2-éthylhexyle, le diisopropylcinnamate de méthyle, l'anthranilate de menthyle, les dérivés de benzotriazole, les dérivés de triazine, les dérivés de β,β'-diphénylacrylate tels que le 2-cyano-3,3-diphénylacrylate de 2-éthylhexyle et le 2-cyano-3,3-diphénylacrylate d'éthyle, l'acide 2-phénylbenzimidazole-5-sulfonique et ses sels, les dérivés de benzophénone, les dérivés du benzylidène-camphre, les filtres siliconés, etc..

A titre d'antioxydants et d'agents anti-radicaux libres utilisables dans la présente invention, on peut citer, par exemple, l'acide ascorbique, des composés ascorbylés tels que le dipalmitate d'ascorbyle, la t-butylhydroquinone, les polyphénols tels que le phloroglucinol, le sulfite de sodium, l'acide érythorbique, les flavonoïdes.

Les chélatants peuvent être choisis notamment parmi l'EDTA (acide éthylènediaminetétraacétique) et ses sels tels que l'EDTA disodique et l'EDTA dipotassique, les composés phosphatés tels que le métaphosphate de sodium, l'hexamétaphosphate de sodium, le pyrophosphate tétrapotassique, les acides phosphoniques et leurs sels tels que les sels de l'acide éthylènediaminetétraméthylènephosphonique.

Les agents antipelliculaires sont choisis, par exemple, parmi :
- le chlorure de benzéthonium, le chlorure de benzalkonium, la chlorhexidine, la chloramine T, la chloramine B, la 1,3-dibromo-5,5-diméthylhydantoïne, la 1,3-dichloro 5,5-diméthylhydantoïne, la 3-bromo 1-chloro 5,5-diméthylhydantoïne, le N-chlorosuccinimide ;
- les dérivés de 1-hydroxy-2-pyridone tels que, par exemple, le 1-hydroxy-4-méthyl-2-pyridone, le 1-hydroxy-6-méthyl-2-pyridone et le 1-hydroxy-4,6-diméthyl-2-pyridone ;
- les trihalogénocarbamides ;
- le triclosan ;
- les composés azolés tels que le climbazole, le kétoconazole, le clotrinazole, l'econazole, l'isoconazole et le miconazole b ;
- les polymères antifongiques tels que l'amphotéricine B ou la nystatine ;
- les sulfures de sélénium ;
- le soufre sous ses différentes formes, le sulfure de cadmium, l'allantoïne, les goudrons de houille ou de bois et leurs dérivés en particulier, l'huile de cade, l'acide undécylénique, l'acide fumarique, les allylamines telles que la terbinafine.

On peut également les utiliser sous forme de leurs sels d'addition d'acides physiologiquement acceptables, notamment sous forme de sels d'acides sulfurique, nitrique, thiocyanique, chlorhydrique, bromhydrique, iodhydrique, phosphorique, acétique, benzoïque, glycolique, acéturique, succinique, nicotinique, tartrique, maléique, palmitique, méthane sulfonique, propanoïque, 2-oxopropanoïque, propanedioïque, 2-hydroxy-1,4-butanedioïque, 3-phényl-2-propènoïque, α-hydroxybenzèneacétique, éthanesulfonique, 2-hydroxyéthanesulfonique, 4-méthylbenzènesulfonique, 4-amino-2-hydroxybenzoïque, 2-phénoxybenzoïque, 2-acétyloxybenzoïque, picrique, lactique, citrique, malique, oxalique et d'aminoacides.

Les agents antipelliculaires mentionnés ci-dessus peuvent aussi le cas échéant être utilisés sous forme de leurs sels d'addition de bases organiques ou inorganiques physiologiquement acceptables. Des exemples de bases organiques sont notamment les alcanolamines de faibles poids moléculaires telles que l'éthanolamine, la diéthanolamine, la N-éthyléthanolamine, la triéthanolamine, le diéthylaminoéthanol, le 2-amino-2-méthylpropanedione; les bases non-volatiles telles que l'éthylènediamine, l'hexaméthylènediamine, la cyclohexylamine, la benzylamine, la N-méthylpipérazine; les hydroxydes d'ammonium quaternaires, par exemple, le triméthylbenzyl hydroxyde ; la guanidine et ses dérivés, et particulièrement ses dérivés alkylés. Des exemples de bases inorganiques sont notamment les sels de métaux alcalins, comme le sodium ou potassium; les sels d'ammonium, les sels de métaux alcalino-terreux, comme le magnésium, calcium ; les sels de métaux di, tri ou tétravalents cationiques, comme le zinc, l'aluminium et le zirconium. Les alcanolamines, l'éthylènediamine et les bases inorganiques telles que les sels de métaux alcalins sont préférées.

Les agents régulateurs de séborrhée sont par exemple le succinylchitosane et la poly-β-alanine.

Les agents apaisants sont par exemple l'azulène et l'acide glycyrrhétinique.

Les tensioactifs cationiques sont ceux bien connus en soi, tels que les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyl-trialkylammo-nium, de trialkylbenzylammonium, de trialkylhydroxy-alkylammonium ou d'alkylpyridinium ; les dérivés d'imidazoline.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyltriméthylammonium ou de diméthyldiallylammo-nium ;
(4) les polysaccharides cationiques décrits plus particulièrement dans les brevets US 3 589 578 et 4 031 307 tels que les gommes de guar contenant des groupements cationiques trialkylammonium ;
(5) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(6) les polyaminoamides solubles dans l'eau, tels que ceux notamment décrits dans les brevets français 2 252 840 et 2 368 508 ;
(7) les dérivés de polyaminoamides, par exemple, les polymères acide adipique/diakylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347 ;
(9) les cyclopolymères d'alkyldiallylamine ou de dialkyldiallylammo-nium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874.870, 4 001.432, 3 929 990, 3 966 904, 4 005 193, 4 025 617, 4 025 627, 4 025 653, 4 026 945 et 4 027 020 ;
(11) les polymères de polyammonium quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat® FC 905, FC 550 et FC 370 par la société B.A.S.F ;
(13) les polyamines comme le Polyquart® H vendu par HENKEL, référencées sous le nom de « POLYETHYLENEGLYCOL (15) TALLOW POLYAMINE » dans le dictionnaire CTFA ;
(14) les polymères réticulés de sels de méthacryloyloxyalkyl(C₁-C₄) trialkyl(C₁-C₄)ammonium tels que ceux commercialisés sous le nom de SALCARE® SC 92, SALCARE® SC 95 et SALCARE® SC 96 par la Société ALLIED COLLOIDS ; et
leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les polymères amphotères utilisables conformément à l'invention peuvent être choisis parmi les polymères comportant des motifs B et C répartis statistiquement dans 1a chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium/chlorure d'acrylamidopropyltriméthylammonium vendu sous la dénomination POLYQUART® KE 3033 par la Société HENKEL.
   Le composé vinylique peut être également un sel de dialkyldiallylammonium tel que le chlorure de diéthyldiallylammonium.
   Les copolymères d'acide acrylique et de ce dernier monomère sont proposés sous les appellations MERQUAT® 280, MERQUAT® 295 et MERQUAT® PLUS 3330 par la société CALGON.
(2) les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

   Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide. le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique. Les comonomères basiques préférés sont des méthacrylates d'aminoéthyle, de butylaminoéthyle, de N,N'-diméthylaminoéthyle, de N-tertio-butylaminoéthyle. On utilise particulièrement les copolymères dont la dénomination CTFA (4ème Ed, 1991) est Octylacrylamide/acrylates/butylaminoethylmethacrylate copolymer tels que les produits vendus sous la dénomination AMPHOMER® ou LOVOCRYL® 47 par la société NATIONAL STARCH.
(3) les polyaminoamides réticulés et alkylés partiellement ou totalement dérivant de polyaminoamides de formule générale : 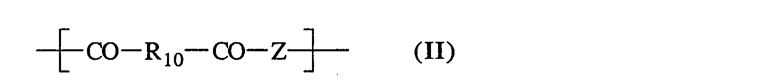 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 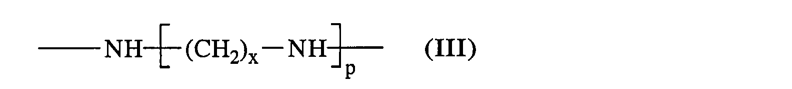 où x=2 et p=2 ou 3, ou bien x=3 et p=2
      ce groupe dérivant de la diéthylène-triamine, de la triéthylènetétraamine ou de la dipropylène-triamine;
   b) dans les proportions de 0 à 40 % en moles, le groupe (III) ci-dessus, dans lequel x=2 et p=1 et qui dérive de l'éthylènediamine, ou le groupe dérivant de la pipérazine 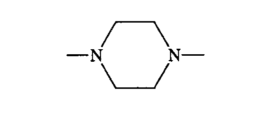
   c) dans les proportions de 0 à 20 % en moles, le groupe -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.
      Les acides carboxyliques saturés sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique. Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 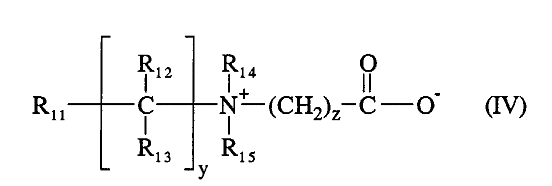 dans laquelle R₁₁ désigne un groupement insaturé polymérisable tel qu'un groupement acrylate, méthacrylate, acrylamide ou méthacrylamide, y et z représentent chacun un nombre entier de 1 à 3, R₁₂ et R₁₃ représentent un atome d'hydrogène, un groupement méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
   A titre d'exemple, on peut citer le copolymère de méthacrylate de méthyle et de diméthyl-carboxyméthylammonio-éthylméthacrylate de méthyle tel que le produit vendu sous la dénomination DIAFORMER® Z301 par la société SANDOZ.
(5) les polymères dérivés du chitosane comportant des motifs monomères répondant aux formules suivantes: 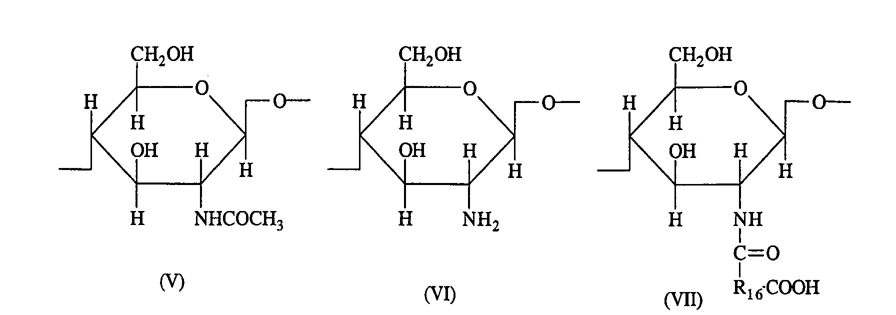 le motif (V) étant présent dans des proportions comprises entre 0 et 30 %, le motif (VI) dans des proportions comprises entre 5 et 50 % et le motif (VII) dans des proportions comprises entre 30 et 90 %, étant entendu que dans ce motif F, R₁₆ représente un groupe de formule: 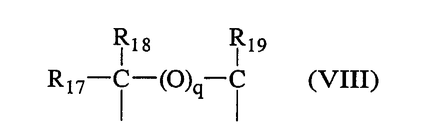 dans laquelle si q=0, R₁₇, R₁₈ et R₁₉, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl-chitosane ou le N-carboxybutylchitosane vendu sous la dénomination EVALSAN® par la société JAN DEKKER.
(7) Les polymères répondant à la formule générale (IX) décrits par exemple, dans le brevet français 1 400 366: 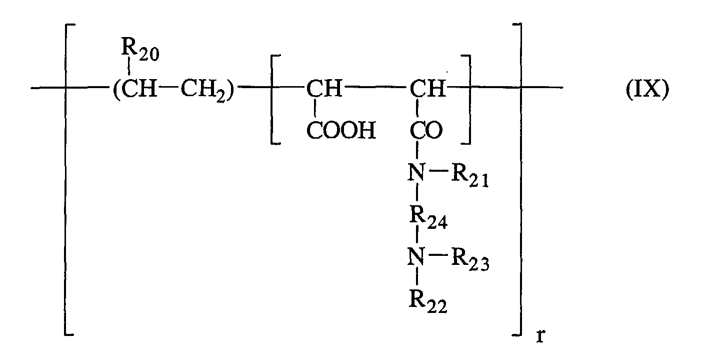 dans laquelle R₂₀ représente un atome d'hydrogène, un groupe CH₃O, CH₃CH₂O, phényle, R₂₁ désigne l'hydrogène ou un groupe alkyle inférieur tel que méthyle, éthyle, R₂₂ désigne l'hydrogène ou un groupe alkyle inférieur tel que méthyle, éthyle, R₂₃ désigne un groupe alkyle inférieur tel que méthyle, éthyle ou un groupe répondant à la formule: -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus,
   ainsi que les homologues supérieurs de ces groupes et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule:

      -D-X-D-X-D- (X)

      où D désigne un groupe 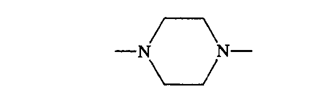 et X désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (XI)

      où D désigne un groupe 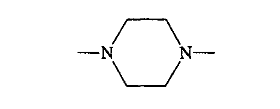 et X désigne le symbole E ou E' et au moins une fois E'; E ayant la signification indiquée ci-dessus et E' est un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther/anhydride maléique modifié partiellement par semiamidification avec une N,N-dialkylaminoalkylamine telle que la N,N-diméthylaminopropylamine ou par semiestérification avec une N,N-dialcanolamine Ces copolymères peuvent également comporter d'autres comonomères vinyliques tels que le vinylcaprolactarne.

Les silicones utilisables conformément à l'invention peuvent être solubles ou insolubles dans l'eau, et elles peuvent être en particulier des polyorganosiloxanes insolubles dans l'eau; elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 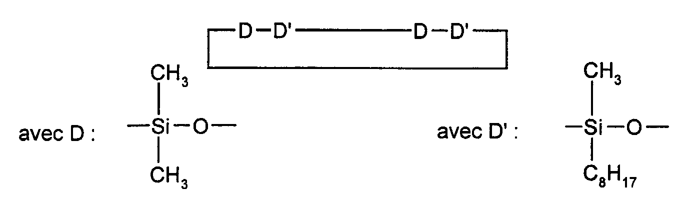
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAT SILICONE. Des silicones entrant dans cette classe sont également décrites dans l'article publié dans Cosmetics and toiletries, Vol. 91, Jan. 76, p. 27-32 - TODD & BTERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SIL WET® L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLTMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 .
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkylcarboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255".
- des groupements hydroxyacylamino, comme les polyorganosiloxanes décrits dans la demande EP 342 834. On peut citer, par exemple, le produit Q2-8413 de la société DOW CORNING.

On peut notamment citer comme huiles d'origine végétale, l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; comme huile d'origine animale, le perhydrosqualène ; comme huiles d'origine minérale, l'huile de paraffine et l'huile de vaseline.

Les polyisobutènes et poly(α-oléfines) sont choisis parmi ceux bien connus dans la technique.

Comme exemples d'esters, on peut notamment citer les esters d'acides gras comme le myristate d'isopropyle, le palmitate d'isopropyle, le palmitate de 2-éthylhexyle, l'huile de Purcellin (octanoate de stéaryle), l'isononanoate d'isononyle ou d'isostéaryle, le lanolate d'isopropyle, et leurs mélanges.

Les polymères anioniques généralement utilisés dans la présente invention sont des polymères comportant des groupes dérivés d'acides carboxylique, sulfonique ou phosphorique, et présentant une masse moléculaire en poids comprise entre 500 et 5 000 000.

Les groupements carboxyliques sont apportés par des monomères monoacides ou diacides carboxyliques insaturés tels que ceux répondant à la formule : 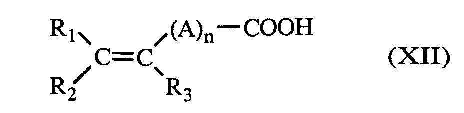 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂-COOH, phényle ou benzyle.

Dans la formule (XII) ci-dessus, un groupement alkyle inférieur comporte de préférence de 1 à 4 atomes de carbone et désigne en particulier, les groupements méthyle et éthyle.

Les polymères anioniques à groupements carboxyliques préférés selon l'invention sont :
A) les homo- ou copolymères d'acide acrylique ou méthacrylique ou leurs sels, et en particulier les produits vendus sous les dénominations VERSICOL® E ou K par la société ALLIED COLLOID, ULTRAHOLD® par la société BASF Les copolymères d'acide acrylique et d'acrylamide vendus sous la forme de leur sel de sodium sous les dénominations RETEN® 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER® par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER® MAEX par la société BASF.
C) les copolymères dérivés d'acide crotonique tels que ceux comportant dans leur chaîne des motifs acétate ou propionate de vinyle, et éventuellement d'autres monomères tels que les ester allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ® AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.

Les polymères comprenant les groupements sulfoniques sont des polymères comportant des motifs vinylsulfonique, styrènesulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinyipyrrolidone ;
- les sels de l'acide polystyrène-sulfonique, les sels de sodium, ayant une masse moléculaire d'environ 500 000 et d'environ 100 000 vendus respectivement sous les dénominations Flexan® 500 et Flexan® 130 par National Starch. Ces composés sont décrits dans le brevet FR 2198719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER® HSP 1180 par Henkel.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG® par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vynyliques, des halogénures vinyliques, des dérivés phénylvinyliques, l'acide acrylique et ses esters tels que le copolymère méthylvinyléther/anhydride maléïque monoestérifié vendu sous la dénomination GANTREZ® ES 425 par la société ISP, les copolymères d'acide méthacrylique et de méthacrylate de méthyle vendus sous la dénomination EUDRAGIT® L par la société ROHM PHARMA, le copolymère d'acide méthacrylique et d'acrylate d'éthyle vendu sous la dénomination LUVIMER® MAEX par la société BASF, le copolymère acétate de vinyle/acide crotonique vendu sous la dénomination LUVISET® CA 66 par la société BASF et le terpolymère acétate de vinyle/acide crotonique/polyéthylèneglycol sous la dénomination ARISTOFLEX® A par la société BASF.

Selon l'invention, on peut également utiliser les polymères anioniques sous forme de latex ou de pseudolatex, c'est-à-dire sous forme d'une dispersion aqueuse de particules de polymères insolubles.

A titre de polymères non ioniques utilisables selon la présente invention, on peut notamment citer :
- les homopolymères de vinyipyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX® 50 000, PEOX® 200 000 et PEOX® 500 000 ;
- les homopolymères d'acétate de vinyle tels que le produit proposé sous le nom de APPRETAN® EM par la société HOECHST ou le produit proposé sous le nom de RHODOPAS® A 012 par la société RHONE POULENC ;
- les copolymères d'acétate de vinyle et d'ester acrylique tels que le produit proposé sous le nom de RHODOPAS® AD 310 de RHONE POULENC ;
- les copolymères d'acétate de vinyle et d'éthylène comme le produit proposé sous le nom de APPRETAN® TV par la société HOECHST;
- les copolymères d'acétate de vinyle et d'ester maléique, par exemple de maléate de dibutyle tels que le produit proposé sous le nom de APPRETAN® MB EXTRA par la société HOECHST ;
- les copolymères de polyéthylène et d'anhydride maléique ;
- les homopolymères d'acrylates d'alkyle et les homopolymères de méthacrylates d'alkyle tels que le produit proposé sous la dénomination MICROPEARL® RQ 750 par la société MATSUMOTO ou le produit proposé sous la dénomination LUHTDRAN® A 848 S par la société BASF ;
- les copolymères d'esters acryliques tels que, par exemple, les copolymères d'acrylates d'alkyle et de méthacrylates d'alkyle comme les produits proposés par la société ROHM & HAAS sous les dénominations PRIMAL® AC-261 K et EUDRAGIT® NE 30 D, par la société BASF sous les dénominations ACRONAL® 601, LUHYDRAN® LR 8833 ou 8845, par la société HOECHST sous les dénominations APPRETAN® N 9213 ou N921 2 ;
- les copolymères d'acrylonitrile et d'un monomère non ionique choisi, par exemple, parmi le butadiène et les (méth)acrylates d'alkyle; on peut citer les produits proposés sous les dénominations NIPOL® LX 531 8 par la société NIPPON ZEON ou ceux proposés sous la dénomination CJ 0601 8 par la société ROHM & HAAS ;
- les polyuréthannes tels que les produits proposés sous les dénominations ACRYSOL® RM 1020 ou ACRTSOL® RM 2020 par la société ROHM & HAAS, les produits URAFLEX® XP 401 UZ, URAFLEX® XP 402 UZ par la société DSM RESINS ;
- les copolymères d'acétate d'alkyle et d'uréthanne tels que le produit 8538-33 par la société NATIONAL STARCH ;
- les polyamides tels que le produit ESTAPOR® LO 11 proposé par la société RHONE POULENC ;
- les gommes de guar non ioniques chimiquement modifiées ou non modifiées.

Les gommes de guar non ioniques non modifiées sont, par exemple, les produits vendus sous la dénomination VIDOGUM® GH 175 par la société UNIPECTINE et sous la dénomination JAGUAR® C par la société MEYHALL.

Les gommes de guar non-ioniques modifiées, utilisables selon l'invention, sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple, être préparées en faisant réagir des oxydes d'alcènes correspondants, tels que, par exemple, des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont, par exemple, vendues sous les dénominations commerciales JAGUAR® HP8, JAGUAR® HP60 et JAGUAR® HP120, JAGUAR® DC 293 et JAGUAR® HP 105 par la société MEYHALL, ou sous la dénomination GALACTASOL® 4H4FD2 par la société AQUALON.

Les groupes alkyle des polymères non ioniques comportent de préférence de 1 à 6 atomes de carbone.

Les agents réducteurs peuvent être choisis parmi les thioacides et leurs sels (acide thioglycolique ou thiolactique, cystéine ou cystéamine), les sulfites de métaux alcalins ou alcalinoterreux, les sucres réducteurs tels que le glucose, la vitamine C et ses dérivés, les dérivés d'acide sulfinique, les phosphines.

Par particules, on englobe les particules minérales et les particules organiques. Les particules minérales peuvent être constituées par les oxydes, les oxydes dihydrates, les hydroxydes, les carbonates, les sulfures, les silicates, les phosphates de silicium, de calcium, de magnésium, de zinc, d'aluminium, de titane, de zirconium, de cerium, les nacres, les micas, les particules constituées par des métaux natifs, c'est-à-dire les métaux alcalins, les métaux alcalinoterreux, les métaux de transitions, les métaux de terres rares, enrobés ou non et leurs alliages. Parmi les métaux, sont préférés l'aluminium, le cuivre, l'argent, l'or, l'indium, le fer, le platine, le nickel, le molybdène, le silicium, le titane, le tungstène, l'antimoine, le palladium, le cadnium, le zinc, l'étain, le sélénium et leurs alliages. Parmi la précédente liste sont préférés l'or, l'argent, le palladium et le platine et leurs alliages, l'argent et l'or étant les métaux préférés.

Les particules organiques peuvent être constituées par les latex, le polystyrène, les dérivés de polystyrène, des silicones, des polymères fluorés, le polypropylène, le polyéthylène, l'acide poly(méth)acrylique, le polyméthacrylate, le polyuréthane, le polyamide, le polycarbonate, le chlorure de polyvinyle, l'acétate de polyvinyle, les fluoropolymères, les polyéthylènes, le polypropylène, le polysobutylène, poly(1-butylène), les copolymères et mélanges des polymères cités, l'alkyl cellulose, l'hydroxyalkyle cellulose, les éthers de cellulose, les esters de cellulose, l'hydroxypropylcellulose, l'hydroxypropyldextrane, l'hydroxypropylméthyl cellulose, l'acétate de cellulose, le carboxyéthylcellulose, le sulfate de cellulose, le sulfate de dextrane, l'alcool polyvinylique, le polyéthylène oxide, le chlorure de polyvinyle, le polyvinylpyrolidone.

Les particules de polystyrène sont disponibles commercialement chez Polyscience Inc (Warrington, PA) ou Duke Scientific Corporation (Palo Alto, CA).

Ces particules organiques peuvent être réticulées à l'aide de réticulants comme par exemple, le divinylbenzène, le glutaraldéhyde, le 1,4-bis(acryloyl)pipérazine, les carbodiimides, le N-hydroxysuccinimide et dérivés, la divinylsufone, le dithiobis(succinimidyl)propionate, le N-succinimidyl-3-(2-pyridyldithio)propionate.

On entend par "particules" des particules dont la taille est comprise entre 1 nm et 100 µm, préférentiellement comprise entre 1nm et 1000 nm et encore plus préférentiellement comprises entre 1nm et 50 nm. On entend par "taille de particule" la dimension maximale qu'il est possible de mesurer entre deux points diamétralement opposés de la particule.

Les particules selon l'invention peuvent être de forme sphérique, lamellaire, fibrillaire, ou de forme totalement aléatoire.

Les composés b) sont de préférence choisis parmi des composés de structure:

D(A' )n'(X) (II)

dans laquelle X désigne (α')m' ou (CW')m")m"(α')p';
- W', A', n', m', m" et p' ayant les mêmes significations possibles que respectivement W, A, n, m et p dans la formule **(I)**,
- α' étant un groupement d'ancrage
- C étant une entité de fixation du résidu W' à l'élément actif D de complexation, D étant tel que l'ensemble D(A' )n'(X) puisse former avec le ou les composés a) un complexe dont la constante de dissociation est inférieure à 10⁻¹ à la température de 25°C.

De préférence α' est choisi parmi la liste suivante :
thiosulfates ; aldéhydes ; époxides ; alkoxysilane ; silanol ; aziridine ; acétal ; hémi-acétal ; aminal ; hémi-aminal ; oxazine et oxazoline ; oxazinium et oxazolinium ; vinyle et vinyle activé : acrylonitrite, esters acrylique et métacrylique, acide et esters crotonique, acides et esters cinnamique, styrène et dérivés, butadiène, éthers de vinylle, vinylacétone, esters maléiques, vinyl sulfones, maléimides ; cétones et alpha-hydroxycétones, alpha-halocétones ; halogénures d'alkyle ou d'aryle, ou aryalkyle RX (X=I, Br, Cl) ; halogénure de cycle insaturé (cycle carboné ou hétérocycle) RX : chlorotriazine, chloropyrimidine, chlorochinoxaline, chlorobenzotriazole ; halogénure de sulfonyle : RSO₂Cl ou F ;(dans les 3 derniers cas d'autres groupes partants X peuvent être aussi : OSO3H, N(Me)3, SO2Me, OPO3H, SO2Et) ; para-nitrophényl ester ; pentafluorophényl ester siloxane ; halogénosilanes, alcoxysilanes ; halogénoacétates ; hydracides ; difluorodinitrobenzène ; maléimides ; esters N-hydroxysuccinimides ; pyridyldithio ; imidate ; isocyanate et isothiocyanate ; dihydroxyacétone et thiols.

La composition selon l'invention peut contenir un ou plusieurs des composés biotine (a) et agents complexants (b) décrits ci-dessus, en une quantité respective comprise entre 0,001 et 90 % en poids, de préférence entre 0,01 et 25 % en poids, mieux encore entre 0,1 et 20 % en poids, par rapport au poids total de la composition de traitement cosmétique.

Le milieu cosmétiquement acceptable peut être constitué uniquement par de l'eau, ou par un solvant ou mélange de solvants, ou par un mélange d'eau et d'un ou plusieurs solvants cosmétiquement acceptables tel qu'un alcool inférieur en C₁-C₄, comme l'éthanol, l'isopropanol, le tertio-butanol, le n-butanol ; des alkylène-glycols comme le propylèneglycol, des éthers de glycol.

Le pH des compositions selon l'invention est compris entre 4 et 8, de préférence entre 5 et 7.

Les compositions selon l'invention peuvent également contenir des additifs tels que des épaississants polymériques naturels ou synthétiques, anioniques, amphotères, zwitteroniques, non ioniques ou cationiques, associatifs ou non, des épaississants non polymériques comme des acides ou des électrolytes, des tensioactifs anioniques, non ioniques, amphotères, des nacrants, des opacifiants, des parfums, des colorants, des particules minérales ou organiques, des conservateurs, des agents de stabilisation du pH.

L'homme de métier veillera à choisir les éventuels additifs et leur quantité de manière à ce qu'ils ne nuisent pas aux propriétés des compositions de traitement.

Ces additifs sont présents dans la composition selon l'invention en une quantité allant de 0 à 20 % en poids par rapport au poids total de la composition.

Ces compositions peuvent se présenter sous la forme de solutions plus ou moins épaisses, de crèmes, de gels fluides, de sticks ou de mousses. Elles peuvent être conditionnées sous forme d'aérosol.

Un objet de l'invention est un ensemble de procédés de traitement cosmétique des matières kératiniques et en particulier des cheveux.

Ces procédés comprennent le greffage d'au moins un composé a) et/ou au moins un composé b) sur les fibres kératiniques.

Un premier procédé selon l'invention consiste à greffer sur les matières kératiniques un composé a) dans lequel T désigne BW(α)p.

Un deuxième procédé selon l'invention consiste à greffer sur les matières kératiniques un composé b) dans lequel X=(CW')m''(α')p'.

Un troisième procédé selon l'invention consiste à greffer sur les matières kératiniques un composé a) dans lequel T=α.

Un quatrième procédé selon l'invention consiste à greffer sur les matières kératiniques un composé b) dans lequel X=(α')m'.

Un cinquième procédé selon l'invention consiste à greffer sur les matières kératiniques, un composé a) dans lequel T désigne α puis dans un second temps à appliquer un composé b) dans lequel X désigne (CW')m".

Un sixième procédé selon l'invention consiste à greffer sur les matières kératiniques, dans un premier temps un composé b) dans lequel X=(α')m' puis dans un second temps à appliquer un composé a) dans lequel T désigne BW.

Un septième procédé selon l'invention consiste à greffer sur les matières kératiniques, dans un premier temps un composé b) dans lequel X=(CW')m"(α')p' puis dans un second temps à appliquer un composé a) dans lequel T désigne BW.

Un huitième procédé selon l'invention consiste à greffer sur les matières kératiniques, dans un premier temps un composé a) dans lequel T désigne BW(α)p puis dans un second temps à appliquer un composé b) dans lequel X désigne (CW')m".

Un neuvième procédé selon l'invention consiste à greffer sur les matières kératiniques, dans un premier temps un composé a) dans lequel T désigne α puis dans un second temps à appliquer un composé b) dans lequel X désigne (CW')m"(α')p'.

Un dixième procédé selon l'invention consiste à greffer sur les matières kératiniques, dans un premier temps un composé b) dans lequel X désigne (CW')m"(α')p' puis dans un second temps à appliquer un composé a) dans lequel T désigne α..

Un onzième procédé selon l'invention consiste à greffer sur les matières kératiniques, dans un premier temps un composé a) dans lequel T désigne BW(α)p puis dans un second temps à appliquer un composé b) dans lequel X désigne (CW')m"(α')p'.

Un douzième procédé selon l'invention consiste à greffer sur les matières kératiniques, dans un premier temps un composé b) dans lequel X désigne (CW')m"(α')p' puis dans un second temps à appliquer un composé a) dans lequel T = BW(α.)p.

Un treizième procédé selon l'invention consiste à greffer sur les matières kératiniques, dans un premier temps un composé b) dans lequel X désigne (α')m' puis dans un second temps à appliquer un composé a) dans lequel T désigne BW(α.)p.

Un quatorzième procédé selon l'invention consiste à greffer sur les matières kératiniques, dans un premier temps un composé a) dans lequel T désigne BW(α)p puis dans un second temps à appliquer temps un composé b) dans lequel X désigne (α')m'.

Un quinzième procédé selon l'invention consiste à greffer sur les matières kératiniques, dans un premier temps un composé b) dans lequel X désigne (α')m' puis dans un second temps à appliquer un composé a) dans lequel T désigne α.

Un seizième procédé selon l'invention consiste à greffer sur les matières kératiniques, dans un premier temps un composé a) dans lequel T désigne α puis dans un second temps à appliquer un composé b) dans lequel X désigne (α')m'.

Optionnellement pour tous les procédés, il est possible d'amener dans une ou plusieurs étapes supplémentaires, un ou plusieurs composés a) ou b). On peut ainsi former des dépôts multicouches et réticuler.

Dans ces derniers procédés on peut faire également intervenir des composés du type D (avidine, streptavidine...).

Un autre objet de l'invention est constitué par des kits multicompartimentaux comportant au moins un compartiment comprenant le composé a) et au moins un compartiment comprenant le composé b).

Un premier type de kit selon l'invention comprend dans un premier compartiment un composé a) dans lequel T désigne α, et dans un deuxième compartiment un composé b) dans lequel X désigne soit (α')m' soit (CW')m" soit (CW')m"(α')p'.

Un deuxième type de kit selon l'invention comprend dans un premier compartiment un composé b) dans lequel X désigne (α')m', dans un deuxième compartiment un composé a) dans lequel T désigne BW(α)p ou BW.

Un autre kit selon l'invention comprend dans un premier compartiment un composé a) dans lequel T désigne BW, dans un deuxième compartiment un composé b) dans lequel X=(CW')m"(α')p'.

Un autre kit selon l'invention comprend dans un premier compartiment un composé b) dans lequel X désigne (CW')m" et dans un deuxième compartiment un composé a) dans lequel T = BW(α)p.

Un autre kit selon l'invention dans un premier compartiment un composé b) dans lequel X désigne (CW')m"(α')p' et dans un deuxième compartiment un composé a) dans lequel T = BW(α)p.

On peut envisager également des kits à 3, 4, 5 ou 6 compartiments si l'on souhaite former des dépôts multi-couches.

On peut également introduire les ingrédients nécessaires à l'invention à l'intérieur de formulation de traitements capillaires existant.

Le procédé de traitement des cheveux selon l'invention peut comprendre une étape préalable qui consiste en un traitement cosmétique choisi dans le groupe formé par les réducteurs ou oxydants pour la déformation permanente des cheveux, les colorations d'oxydation, décolorations, shampooings ou compositions coiffantes.

Les exemples suivants sont destinés à illustrer l'invention, sans toutefois présenter un caractère limitatif.

### Exemples :

Pour démontrer les propriétés de rémanence de la composition selon l'invention par rapport à une simple adsorption physicochimique, la rémanence d'une enzyme ou de billes de latex colorées sur la fibre capillaire a été comparée avec et sans l'utilisation du couple biotine-avidine.

Pour cela, la TFP-PEO-biotine a été utilisée comme biotine greffable sur la surface du cheveu, la TFP-PEO-biotine étant le N-{3-[2-(2-{3-[5-(2-Oxo-hexahydrothieno[3,4-d]imidazo-4-yl)-pentanoylamino]-propoxy}-ethoxy)-ethoxy]propyl)-succinamic acid 2,3,5,6-tetrafluorophenyl ester (Pierce, réf 21219).

### Exemple 1 : Rémanence aux shampooings d'une composition comportant un complexe avidine-enzyme

### Greffage de biotine sur la surface des cheveux en utilisant de la TFP-PEO-biotine

Dans cet exemple, la TFP-PEO-biotine est greffée sur les cheveux puis le complexe avidine-peroxidase est accroché sur la biotine.

La TFP-PEO-biotine réagit sur les amines de la surface du cheveux.

On prélève, pour chaque échantillon, 5 cheveux (soit environ 10 mg) d'une mèche de cheveux naturels lavée que l'on place dans des flacons de verre silanisés.

On prépare une solution de TFP-PEO-biotine à 1 mg/ml dans un tampon phosphate (pH 7,2). 1 ml de cette solution est mis en contact avec l'échantillon. Pour l'échantillon témoin, 1 ml de solution de tampon phosphate (pH 7,2) est mis en contact avec les cheveux. On agite pendant 2 h à température ambiante.

On réalise 3 rinçages à l'eau (avec 3x2 ml d'eau distillée, en agitant avec un vortex pendant 1 min)

### Adsorption et rémanence d'un complexe avidine-peroxydase sur la surface des cheveux:

On prépare ensuite une solution d'avidine-peroxydase (Sigma, réf : A3151) à 50 µg/ml dans le tampon phosphate (pH 7,2). Les cheveux sont incubés dans 1 ml de cette solution pendant 1h à température ambiante.

On réalise un rinçage en plusieurs étapes :
3 rinçages avec 1ml d'une solution de shampooing
3 rinçages avec 1ml d'eau distillée

On ajoute ensuite 1 ml d'un solution de substrat de la peroxydase (Sigma, réf : A3219). Ce substrat, incolore en solution, est transformé en substance colorée (jaune) après réaction avec la peroxidase. Cette substance absorbe fortement en lumière UV (405nm) et permet ainsi de détecter la présence de peroxidase fixée sur le cheveu.

On laisse réagir 5 min puis on stoppe la réaction avec 1 ml d'une solution de SDS 1% (sodiumdodecylsulfate).

On prélève 200µl du surnageant de chaque échantillon, que l'on dépose, après dilution, dans les puits d'une plaque 96 puits. On réalise une lecture spectrophotométrie à 405 nm.

### Résultats :

La densité optique moyenne de la solution obtenue pour les cheveux traités avec la composition selon l'invention est de 1,16.

La densité optique moyenne de la solution obtenue pour les cheveux témoins est de 0,65.

Ces résultats montrent que la TFP-PEO biotine greffée de façon covalente sur le cheveu et résistante aux shampooings augmente la rémanence du complexe avidine-peroxidase par rapport aux cheveux sans biotine.

### Exemple 2 : Rémanence aux shampooings de billes de latex colorées

Dans cet exemple, la TFP-PEO-biotine est greffée sur les cheveu et les billes de latex préalablement enduites d'avidine sont accrochées sur la biotine.

La TFP-PEO-biotine est greffée sur le cheveu selon le protocole décrit précédemment.

### Préparation des billes de latex dérivatisées avec l'avidine

200 µl d'une suspension dans l'eau de billes en polystyrène colorées bleues (Merck) sont placées dans un eppendorf. 1 ml de tampon phosphate (pH 7,2) est ajouté, la suspension est agitée au vortex pendant 1 min, centrifugée (10 min à 13200 rpm) et le surnageant est écarté. Cette opération de lavage est répétée 2 fois.

1 ml d'une solution d'avidine à 1 mg/ml dans le tampon phosphate (pH 7,2) est ajouté au culot de billes de latex. Cette solution est passée au sonicateur puis agitée pendant 1h à température ambiante. Les billes de latex sont centrifugées 10 min à 13200 rpm (rotation par minute), le surnageant est écarté et le culot est ensuite lavé 6 fois avec 1 ml de tampon phosphate comme précédemment décrit. Le culot est suspendu dans 1 ml de ce même tampon et conservé à 4°C.

### Adsorption des billes de latex sur les cheveux

500 µl de la suspension de billes de latex diluées avec 500 µl de tampon phosphate (pH 7,2) sont ajoutés à chaque échantillon : traités et témoins.

Après 1 h d'incubation à température ambiante sous agitation, les cheveux sont rincés selon le protocole suivant :
3 rinçages avec 1 ml d'une solution de shampooing
3 rinçages avec 1 ml d'eau distillée.

Les échantillons de cheveux sont observés en microscopie électronique à balayage.

Les résultats obtenus (cf Photographies en MEB) montrent que les cheveux traités avec la TFP-PEO-biotine présentent une forte concentration de billes à leur surface après les shampooings par rapport aux cheveux non traités. Il y a donc une rémanence marquée aux lavages. Par contre, aucune bille de latex n'est visible sur les cheveux témoins.

## Revendications

1. Composition cosmétique comprenant :
un composé biotine a) et un agent complexant b) susceptible de former un complexe avec le composé biotine de constante de dissociation inférieure ou égale à 10⁻¹ à la température de 25C°, le composé biotine étant lié à un groupement d'ancrage α et/ou l'agent complexant étant lié à un groupement d'ancrage α', les groupements α et α' permettant une fixation covalente aux cheveux et le composé biotine et/ou l'agent complexant étant porteur d'au moins un groupement à activité cosmétique.

2. Composition selon la revendication 1, **caractérisée par le fait que** le composé a) est choisi parmi les composés de formule **(I)** 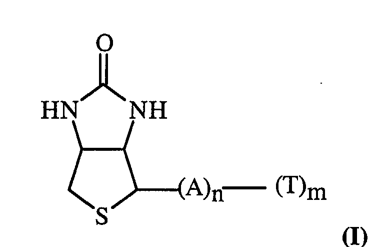
• avec A désignant un radical divalent comprenant de 1 à 100 atomes de carbone, linéaire ou ramifié, saturé ou non, interrompu ou non par un ou plusieurs hétéroatomes et éventuellement substitué par un ou plusieurs groupements choisis parmi les groupements hydroxyle, amino, halogènes, aryle, phosphate, phosphorate, sulfate, sulfonate, carboxy, alcoxycarbonyle, alcoxy.
• avec T désignant un groupement α ou un radical BW(α)ₚ
• W étant un résidu à activité cosmétique et B une entité de fixation du résidu W au squelette dérivé de biotine,
• α désignant un groupement d'ancrage permettant une fixation covalente aux cheveux
• avec n désignant 0 ou 1
• avec m désignant un nombre allant de 1 à 100
• avec p désignant un nombre allant de 0 à 100

3. Composition selon la revendication 2 **caractérisée par le fait que** B désigne un groupement amine, imine, amide, ester, disulfure, thioester, uréthane, urée, éther, thioéther, azoïque, méthine, époxy, un résidu aromatique ou hétérocyclique, dialkylpolysiloxane,

4. Composition selon les revendications 2 ou 3 **caractérisée par le fait que** W est issu de molécules filtrant les UV, de molécules hydratantes ou émollientes, d'agents conditionneurs, d'agents antistatiques, d'agents antitranspirants, de matières parfumantes, de molécules réductrices, de molécules oxydantes, d'agents antimicrobiens, d'agents antipelliculaires, d'agents particulaires minéraux ou organiques sur lesquels sont éventuellement adsorbé un ou plusieurs polymères, d'agents filmogènes anioniques ou non ioniques, amphotères ou cationiques sur lesquels sont éventuellement adsorbées des particules organiques ou minérales.

5. Composition selon l'une quelconque des revendications 2 à 4 **caractérisée par le fait que** dans le composé a), le groupement d'ancrage α est choisi parmi les groupements suivants : thiosulfates ; aldéhydes ; époxides ; alkoxysilane ; silanol ; aziridine ; acétal ; hémi-acétal ; aminal ; hémi-aminal ; oxazine et oxazoline ; oxazinium et oxazolinium ; vinyle et vinyle activé : acrylonitrite, esters acrylique et métacrylique, acide et esters crotonique, acides et esters cinnamique, styrène et dérivés, butadiène, éthers de vinylle, vinylacétone, esters maléiques, vinyl sulfones, maléimides ; cétones et alpha-hydroxycétones, alpha-halocétones ; halogénures d'alkyle ou d'aryle, ou aryalkyle RX (X=I, Br, Cl) ; halogénure de cycle insaturé (cycle carboné ou hétérocycle) RX : chlorotriazine, chloropyrimidine, chlorochinoxaline, chlorobenzotriazole ; halogénure de sulfonyle : RSO₂Cl ou F ;(dans les 3 derniers cas d'autres groupes partants X peuvent être aussi : OSO3H, N(Me)3, SO2Me, OPO3H, SO2Et) ; para-nitrophényl ester ; siloxane ; halogénosilanes, alcoxysilanes ; halogénoacétates ; hydracides ; difluorodinitrobenzène.

6. Composition selon la revendication 1, **caractérisée par le fait que** le composé b) est choisi parmi des composés de formule **(II)**:
D(A' )n'(X) (II)
dans laquelle :
• D étant tel que l'ensemble D(A' )n'(X) puisse former avec le ou les composés a) un complexe dont la constante de dissociation est inférieure à 10-1 à la température de 25°C.
• X désigne (α')_{m'} ou (CW')_{m"}(α')_{p'};
• W', A', n', m', m" et p' ayant les mêmes significations possibles que respectivement W, A et n, m et p dans la formule (I) et C étant une entité de fixation du résidu W à l'élément actif D de complexation,
• α' désignant un groupement d'ancrage permettant une fixation covalente aux cheveux

7. Composition cosmétique selon la revendication 6 **caractérisée par le fait que** dans le composé II le groupement d'ancrage α' est choisi parmi les groupements suivants : thiosulfates ; aldéhydes ; époxides ; alkoxysilane ; silanol ; aziridine ; acétal ; hémi-acétal ; aminal ; hémi-aminal ; oxazine et oxazoline ; oxazinium et oxazolinium ; vinyle et vinyle activé : acrylonitrite, esters acrylique et métacrylique, acide et esters crotonique, acides et esters cinnamique, styrène et dérivés, butadiène, éthers de vinylle, vinylacétone, esters maléiques, vinyl sulfones, maléimides ; cétones et alpha-hydroxycétones, alpha-halocétones ; halogénures d'alkyle ou d'aryle, ou aryalkyle RX (X=I, Br, Cl) ; halogénure de cycle insaturé (cycle carboné ou hétérocycle) RX : chlorotriazine, chloropyrimidine, chlorochinoxaline, chlorobenzotriazole ; halogénure de sulfonyle : RSO₂Cl ou F ;(dans les 3 derniers cas d'autres groupes partants X peuvent être aussi : OSO3H, N(Me)3, SO2Me, OPO3H, SO2Et) ; para-nitrophényl ester ; pentafluorophényl ester siloxane ; halogénosilanes, alcoxysilanes ; halogénoacétates ; hydracides ; difluorodinitrobenzène ; maléimides ; esters N-hydroxysuccinimides ; pyridyldithio ; imidate ; isocyanate et isothiocyanate ; dihydroxyacétone et thiols.

8. Composition cosmétique selon l'une quelconque des revendications 1 à 5 **caractérisée par le fait que** le composé biotine porteur d'au moins un groupement d'ancrage α permettant une fixation covalente aux cheveux, ce groupement d'ancrage α choisi parmi :
thiosulfates ; aldéhydes ; époxides ; alkoxysilane ; silanol ; aziridine ; acétal ; hémi-acétal ; aminal ; hémi-aminal ; oxazine et oxazoline ; oxazinium et oxazolinium ; vinyle et vinyle activé : acrylonitrite, esters acrylique et métacrylique, acide et esters crotonique, acides et esters cinnamique, styrène et dérivés, butadiène, éthers de vinylle, vinylacétone, esters maléiques, vinyl sulfones, maléimides ; cétones et alpha-hydroxycétones, alpha-halocétones ; halogénures d'alkyle ou d'aryle, ou aryalkyle RX (X=I, Br, Cl) ; halogénure de cycle insaturé (cycle carboné ou hétérocycle) RX :chlorotriazine, chloropyrimidine, chlorochinoxaline, chlorobenzotriazole ; halogénure de sulfonyle : RSO₂Cl ou F ;(dans les 3 derniers cas d'autres groupes portants X peuvent être aussi : OSO3H, N(Me)3, SO2Me, OPO3H, SO2Et) para-nitrophényl ester ; siloxane ; halogénosilanes, alcoxysilanes ; halogénoacétates ; hydracides ; difluorodinitrobenzène.

9. Composition cosmétique selon la revendication 8 **caractérisée par le fait qu'**elle comprend au moins un composé a) de formule (I) avec T désignant α.

10. Composition cosmétique selon l'une quelconque des revendications 1 à 5 **caractérisée par le fait qu'**elle comprend au moins un composé biotine porteur d'au moins un groupement d'ancrage α permettant une fixation covalente aux cheveux et le composé biotine étant également porteur d'au moins un groupement à activité cosmétique.

11. Composition cosmétique selon la revendication 10 **caractérisée par le fait qu'**elle comprend au moins un composé a) de formule (I) avec T désignantBW(α)p.

12. Composition cosmétique selon l'une quelconque des revendications 1 et 6 et 7 **caractérisée par le fait qu'**elle comprend au moins un agent complexant du composé biotine (biotine modifiée ou non), porteur d'au moins un groupement d'ancrage α' permettant une fixation covalente aux cheveux.

13. Composition cosmétique selon la revendication 12 **caractérisée par le fait qu'**elle comprend au moins un composé b) de formule (II) avec X désignant (α')m'.

14. Composition cosmétique selon l'une quelconque ees revendications 1 et 6 et 7 **caractérisée par le fait qu'**elle comprend au moins un agent complexant du composé biotine (biotine modifiée ou non), porteur d'au moins un groupement d'ancrage α' permettant une fixation covalente aux cheveux et l'agent complexant étant également porteur d'au moins un groupement à activité cosmétique.

15. Composition cosmétique selon la revendication 14 **caractérisée par le fait qu'**elle comprend au moins un composé b) de formule (II) avec X désignant (CW')m"(α')p'.

16. Composition selon l'une quelconque des revendications 1 à 15, **caractérisée par le fait qu'**elle se présente sous la forme d'une solution aqueuse, alcoolique ou hydroalcoolique plus ou moins épaisse, d'une crème, d'un gel fluide, d'un stick, d'une mousse, éventuellement conditionnés sous forme aérosol.

17. Procédé de traitement cosmétique de matières kératiniques **caractérisé par le fait que** l'on greffe sur les matières kératiniques au moins un composé a) et/ou au moins un composé b) tel que défini dans la revendication 1.

18. Procédé de traitement cosmétique des matières kératiniques et en particulier des cheveux selon la revendication 17, consistant à greffer sur les matières kératiniques un composé a) selon l'une quelconque des revendications 2 à 5 dans lequel T désigne BW(α)p.

19. Procédé de traitement cosmétique des matières kératiniques et en particulier des cheveux selon la revendication 17, consistant à greffer sur les matières kératiniques un composé b) selon l'une quelconque des revendications 6 et 7 dans lequel X=(CW')m''(α')p'.

20. Procédé de traitement cosmétique des matières kératiniques et en particulier des cheveux selon la revendication 17, consistant à greffer sur les matières kératiniques un composé a) selon l'une quelconque des revendications 2 et 5 dans lequel T=α.

21. Procédé de traitement cosmétique des matières kératiniques et en particulier des cheveux selon la revendication 17, consistant à greffer sur les matières kératiniques un composé b) selon l'une quelconque des revendications 6 et 7 dans lequel X=(α')m'.

22. Procédé de traitement cosmétique des matières kératiniques et en particulier des cheveux selon la revendication 17, consistant à greffer sur les matières kératiniques un composé a) selon l'une quelconque des revendications 2 et 5 dans lequel T désigne α puis dans un second temps à appliquer un composé b) selon l'une quelconque des revendications 6 et 7 dans lequel X désigne (CW')m".

23. Procédé de traitement cosmétique des matières kératiniques et en particulier des cheveux selon la revendication 17, consistant à greffer sur les matières kératiniques dans un premier temps un composé b) selon l'une quelconque des revendications 6 et 7 dans lequel X=(α')m' puis dans un second temps à appliquer un composé a) selon l'une quelconque des revendications 2 et 5 dans lequel T désigne BW.

24. Procédé de traitement cosmétique des matières kératiniques et en particulier des cheveux selon la revendiction 17, consistant à greffer sur les matières kératiniques dans un premier temps un composé b) selon l'une quelconque des revendications 6 et 7 dans lequel X=(CW')m"(α')p' puis dans un second temps à appliquer un composé a) selon l'une quelconque des revendications 2 et 5 dans lequel T désigne BW.

25. Procédé de traitement cosmétique des matières kératiniques et en particulier des cheveux selon la revendication 17, consistant à greffer sur les matières kératiniques dans un premier temps un composé a) selon l'une quelconque des revendications 2 et 5 dans lequel T désigne BW(α)p puis dans un second temps à appliquer un composé b) selon l'une quelconque des revendications 6 et 7 dans lequel X désigne (CW')_{m''}.

26. Procédé de traitement cosmétique des matières kératiniques et en particulier des cheveux selon la revendication 17, consistant à greffer sur les matières kératiniques dans un premier temps un composé a) selon l'une quelconque des revendications 2 et 5 dans lequel T désigne α puis dans un second temps à appliquer un composé b) selon l'une quelconque des revendications 6 et 7 dans lequel X désigne (CW')m"(α')p'.

27. Procédé de traitement cosmétique des matières kératiniques et en particulier des cheveux selon la revendication 17, consistant à greffer sur les matières kératiniques dans un premier temps un composé b) selon l'une quelconque des revendications 6 et 7 dans lequel X désigne (CW')m"(α')p' puis dans un second temps à appliquer un composé a) selon l'une quelconque des revendications 2 et 5 dans lequel T désigne α.

28. Procédé de traitement cosmétique des matières kératiniques et en particulier des cheveux selon la revendication 17, consistant à greffer sur les matières kératiniques dans un premier temps un composé a) selon l'une quelconque des revendications 2 et 5 dans lequel T désigne BW(α)p puis dans un second temps à appliquer un composé b) selon l'une quelconque des revendications 6 et 7 dans lequel X désigne (CW')m"(α')p'.

29. Procédé de traitement cosmétique des matières kératiniques et en particulier des cheveux selon la revendication 17, consistant à greffer sur les matières kératiniques dans un premier temps un composé b) selon l'une quelconque des revendications 6 et 7 dans lequel X désigne (CW')m"(α')p' puis dans un second temps à appliquer un composé a) selon l'une quelconque des revendications 2 et 5 dans lequel T = BW(α)p.

30. Procédé de traitement cosmétique des matières kératiniques et en particulier des cheveux selon la revendication 17, consistant à greffer sur les matières kératiniques dans un premier temps un composé b) selon l'une quelconque des revendications 6 et 7 dans lequel X désigne (α')m' puis dans un second temps à appliquer un composé a) selon l'une quelconque des revendications 2 et 5 dans lequel T désigne BW(α)p.

31. Procédé de traitement cosmétique des matières kératiniques et en particulier des cheveux selon la revendication 17, consistant à greffer sur les matières kératiniques dans un premier temps un composé a) selon l'une quelconque des revendications 2 et 5 dans lequel T désigne BW(α)p puis dans un second temps à appliquer temps un composé b) selon l'une quelconque des revendications 6 et 7 dans lequel X désigne (α')m'.

32. Procédé de traitement cosmétique des matières kératiniques et en particulier des cheveux selon la revendication 17, consistant à greffer sur les matières kératiniques dans un premier temps un composé b) selon l'une quelconque des revendications 6 et 7 dans lequel X désigne (α')m' puis dans un second temps à appliquer un composé a) selon l'une quelconque des revendications 2 et 5 dans lequel T désigne α.

33. Procédé de traitement cosmétique des matières kératiniques et en particulier des cheveux selon la revendication 17, consistant à greffer sur les matières kératiniques dans un premier temps un composé a) selon l'une quelconque des revendications 2 et 5 dans lequel T désigne α puis dans un second temps à appliquer un composé b) selon l'une quelconque des revendications 6 et 7 dans lequel X désigne (α')m'.

34. Procédé de traitement cosmétique des matières kératiniques selon l'une quelconque des revendications 17 à 33, **caractérisée par le fait que** un ou plusieurs composés a) selon les revendications 2 et 5 ou b) selon les revendications 6 et 7 ou de type D selon la revendication 6 sont déposés dans une ou plusieurs étapes supplémentaires.

35. Kit de traitement cosmétique des matières kératiniques **caractérisé par le fait qu'**il comprend au moins un compartiment comprenant le composé a) et au moins un compartiment comprenant le composé b), a) et b) étant définis comme dans la revendication 1.

36. Kit de traitement des matières kératiniques selon la revendication 35 **caractérisé par le fait qu'**il comprend dans un premier compartiment un composé a) selon l'une quelconque des revendications 2 et 5 dans lequel T désigne α et dans un deuxième compartiment un composé b) selon l'une quelconque des revendications 6 et 7 dans lequel X désigne soit (α')m' soit (CW')m" soit (CW')m"(α')p'.

37. Kit de traitement des matières kératiniques selon la revendication 35 **caractérisé par le fait qu'**il comprend dans un premier compartiment un composé b) selon l'une quelconque des revendications 6 et 7 dans lequel X désigne (α')m' et dans un deuxième compartiment un composé a) selon l'une quelconque des revendications 2 à 5 dans lequel T désigne BW(α)p ou BW.

38. Kit de traitement des matières kératiniques selon la revendication 35 **caractérisé par le fait qu'**il comprend dans un premier compartiment un composé a) selon l'une quelconque des revendications 2 et 5 dans lequel T désigne BW et dans un deuxième compartiment un composé b) selon l'une quelconque des revendications 6 et 7 dans lequel X=(CW')m"(α')p'.

39. Kit de traitement des matières kératiniques selon la revendication 35 **caractérisé par le fait qu'**il comprend dans un premier compartiment un composé b) selon l'une quelconque des revendications 6 et 7 dans lequel X désigne (CW')m" et dans un deuxième compartiment un composé a) selon l'une quelconque des revendications 2 et 5 dans lequel T = BW(α)p.

40. Kit de traitement des matières kératiniques selon la revendication 35 **caractérisé par le fait qu'**il comprend dans un premier compartiment un composé b) selon l'une quelconque des revendications 6 et 7 dans lequel X désigne (CW')m"(α')p' et dans un deuxième compartiment un composé a) selon l'une quelconque des revendications 2 et 5 dans lequel T = BW(α)p.

41. Kit de traitement des matières kératiniques selon l'une quelconque des revendications 35 à 40 **caractérisé par le fait qu'**il comprend 3, 4, 5 ou 6 compartiments.

42. Procédé de traitement des matières kératiniques, **caractérisé par le fait qu'**il comprend une étape préalable consistant en un traitement cosmétique choisi parmi l'application de réducteurs ou oxydants pour la déformation permanente des cheveux, la coloration d'oxydation, la décoloration, un shampooing ou l'application d'une composition coiffante puis que l'on applique en quantité suffisante, une composition cosmétique telle que définie dans l'une quelconque des revendications 1 à 16.
